(19) **Europäisches Patentamt European Patent Office Office européen des brevets**

(11) **EP 4 309 812 A1**

(12) **EUROPEAN PATENT APPLICATION**

(43) Date of publication:
24.01.2024 Bulletin 2024/04

(21) Application number: 23185685.7

(22) Date of filing: 14.07.2023

(51) International Patent Classification (IPC):
**B09B 1/00** (2006.01)

(52) Cooperative Patent Classification (CPC):
**B09B 1/006; B09B 1/004;** C12M 21/04;
C12M 23/36

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 14.07.2022 US 202263389344 P

(71) Applicant: **Watershed Geosynthetics LLC Alpharetta GA 30022 (US)**

(72) Inventors:
• **LEWIS, Delaney**
 **Louisiana, 71234 (US)**
• **URRUTIA, Jose**
 **Georgia, 30024 (US)**

(74) Representative: **Sharman, Thomas Alexander Reddie & Grose LLP The White Chapel Building 10 Whitechapel High Street London E1 8QS (GB)**

(54) **FAIL-SAFE WASTE GAS COLLECTION SYSTEM**

(57) A fail-safe waste gas collection system for a waste pile having a membrane-covered portion and an uncovered portion, the waste pile having a waste gas collection grid extending to both the membrane-covered portion of the waste pile and the uncovered portion of the waste pile, and a vacuum pump connected to the waste gas collection grid. The fail-safe waste gas collection system comprises a plurality of pressure relief valves to help protect a membrane against ballooning in the event that a vacuum pump stops operating; and an automatic isolation valve to isolate a membrane-covered portion of a collection grid from an uncovered portion of a collection grid when a gas pressure in an uncovered portion of a collection grid exceeds a gas pressure in a membrane-covered portion of a collection grid.

fig.5

## Description

TECHNICAL FIELD

The present invention relates generally to landfills or waste piles and, in particular, to a fail-safe system for collecting waste gas from a waste pile. The present invention may be suitable for minimizing a risk of dislodging a membrane positioned over the waste pile in the event of a power failure.

BACKGROUND

Landfills are created incrementally and, as they grow, they do so in a largely linear path. They tend to start at one end of a site and as one initial block or area becomes full, another block or area of landfill is opened up next to it. Over time, the landfill grows much like adding fat/thick slices of a baguette back together to reassemble the baguette.

For many years, landfills were left uncovered. In more recent years, they have been covered with grass or dirt, both of which permit some or all of the gas created by the decomposition of the waste to escape to the atmosphere. It has become common to collect the landfill gas via deep wells connected to a large pump (fan or vacuum source) to pull the landfill gas from the landfill and to burn it at a flare on-site or nearby.

In more recent years, some landfills have begun to be capped or covered with an impermeable membrane. Given how the landfills grow and develop incrementally as described above, it often occurs that some parts of a landfill have an impermeable membrane, while other parts of the landfill do not. In these situations, when the gas collection system is offline (for instance, due to a power outage, mechanical failure, weather event, etc.), pressure differentials in the landfill and/or in the gas collection system can cause the impermeable membranes to "balloon", destabilizing the installation of the membranes and damaging the landfill.

In the past, it has been known to use pumps, piping, and wellheads to extract the gases from the landfill and collect the same. Such wellheads are often spaced about one per 4000m2, approximately, in a grid pattern. Such systems of collecting the gases can be shut down by many factors, including power failures. To prevent the undesirable build-up of such gases in the event of non-operation of the extraction system, it has often been known to employ a grid pattern of vents spaced between the extraction wellheads, often at the same one per 4000m2 density, approximately.

As described in published U.S. Patent Application Number 20060034664, conventional gas extraction wells at landfills often involve deep wells attached to a network of pipes and a gas pump (blower) that applies vacuum (negative pressure) to extract the gas from the stored waste as the waste decomposes.

A prior art deep well arrangement according to the above published patent application is shown in FIG. 1 (prior art). Landfill 1 containing waste W generates biogas (biogas flows shown by the arrows). Biogas is collected and extracted through a well 3. The well 3 includes a gas-collecting well screen 16 and a gas-impermeable conduit 17 linking the well screen to the surface to draw biogas from the wellhead to the surface. Overlaying the majority of the waste W is a gas-permeable layer 5. The term "wellhead" refers to a portion of the gas-extraction well from which gas can be extracted. The well often includes a section of pipe having slots or other gas-flow apertures cut in it, referred to as a "well screen". Often, the well screen is also surrounded with gravel.

The gas-permeable layer is typically composed of a conductive porous matrix with gas flow paths. Often it is composed of rigid or semi-rigid particles of a large enough size to leave a significant void volume between particles. For instance, the gas-permeable layer may contain sand, gravel, wood chips, or shredded tires. Above the gas-permeable layer is a gas-containment layer 7. Biogas that rises from the landfill reaches the gas-permeable layer where it is trapped by the overlying gas-containment layer 7. The biogas migrates horizontally in the gas-permeable layer until it comes near to a well. Gas extraction from the well creates a vacuum that draws gas into the well. This vacuum draws biogas from the overlying gas-permeable layer down through the waste mass of the landfill to reach the well.

Beneath the gas-permeable high conductivity layer 5 through which a substantial fraction of the biogas from the gas-permeable layer passes as it travels to the gas-collection wellhead is an entrainment zone 9. On its passage through the waste 2, the gas from the gas-permeable layer mixes with biogas produced in the waste mass that has not gone through the gas-permeable layer. This helps to give a consistent content to the biogas that is withdrawn from the well. If gas is withdrawn directly from the gas-permeable conductive layer, the gas composition will vary more dramatically over time, sometimes containing a high air content and sometimes not. It is sometimes desirable to place an even more impermeable layer, such as geomembrane 15, directly over the zone of entrainment of gas from the permeable layer that is created by the deep well. Moreover, sometimes the entire landfill is covered with such a membrane.

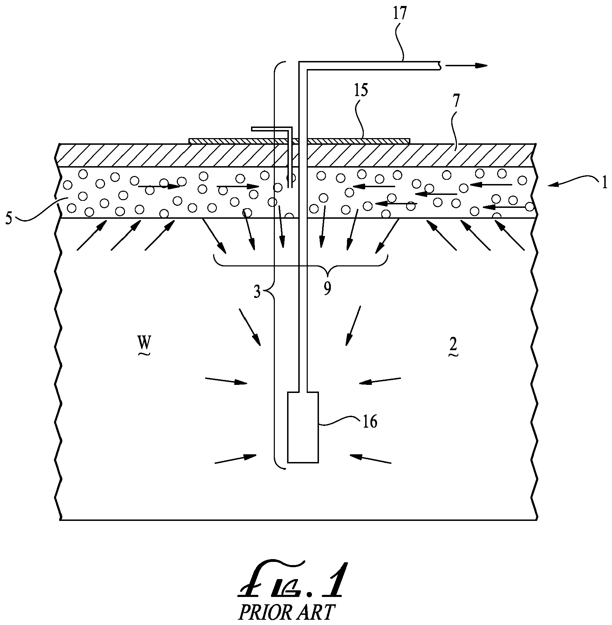

The deep well design of **FIG. 1** (prior art) is designed to pull gas away from the surface to protect the membrane cover from being impacted with gas buildup that can create ballooning. Typically, the deep well has a diminishing radius (zone) of influence as a result of pressure loss through the length of the well collector pipes. The deep well vacuum pressure pulls both gas and leachate into the well. Leachate pumps are often required, resulting in more membrane penetrations. The membrane cover helps alleviate air intrusion issues -- however, multiple penetrations typically are required at each collection point. Membrane penetrations around wellheads are very susceptible to rips and tears, and can result in either gas leaks or air intrusion into the waste. Another drawback to the deep well is that the deep well must be continually monitored and adjusted. Deep wells normally utilize an adjustable valve at each collection point to control pressures within the well to adjust the radius of influence, but have limited maximum radius of influence from the control valve.

**FIG. 2** (prior art) shows another prior art arrangement, this time showing a more shallow wellhead 26 used to withdraw near-surface or sub-surface gas from beneath a membrane **M** capping a waste **W.** The wellhead 26 is attached to an above-ground conduit by way of a vertical pipe.

**FIG. 3** (prior art) shows another prior art arrangement, this time depicting a landfill with multiple wellheads 30 used to withdraw near-surface or sub-surface gas from beneath the surface. The wellheads 30 are attached to an above-ground vent 31.

**FIG. 4** (prior art) shows another prior art arrangement similar to that in **FIG. 2** (prior art), this time showing a field of wellheads 40 spaced to extract the gases from a landfill and collect the same. Such wellheads are often spaced about one per 4000m2, approximately.

One particularly troublesome problem with known prior art gas collection systems for use in landfills is that the systems typically use electrically-powered vacuum pumps to help draw off the waste gas. In the event of a power outage, such as often can occur due to lightning strikes, the waste gas can build up underneath a membrane in the landfill while the electric power is off, resulting in a ballooning effect on the membrane. This ballooning can damage the membrane or even cause it to slide off the side of a waste pile. Accordingly, a need exists for a system that can keep the membrane intact without ballooning in the event of a power outage or failure of the vacuum pump. It is to the provision of such a system that example embodiments of the present invention are at least partially directed.

SUMMARY OF THE INVENTION

Generally described, an example embodiment of the present invention relates to a fail-safe gas collection system for collecting waste gas from a waste pile having a membrane-covered portion and an uncovered portion, the waste pile having a waste gas collection grid extending to both the membrane-covered portion of the waste pile and the uncovered portion of the waste pile, and a vacuum pump connected to the waste gas collection grid, wherein the fail-safe waste gas collection system comprises a plurality of pressure relief valves to help protect a membrane against ballooning in the event that a vacuum pump stops operating; and an automatic isolation valve to isolate a membrane-covered portion of a collection grid from an uncovered portion of a collection grid when a gas pressure in an uncovered portion of a collection grid exceeds a gas pressure in a membrane-covered portion of a collection grid.

Preferably, the automatic isolation valve is operable to isolate the covered portion of the gas collection grid when the pressure in the uncovered portion of the gas collection grid is between about 1 cm and about 80cm of water column head greater in an uncovered portion of a collection grid than a gas pressure in a covered portion of a collection grid. The automatic isolation valve may be operable to isolate the covered portion of the gas collection grid when the pressure in the uncovered portion of the gas collection grid is between about 5cm to about 10cm, or about 6cm to about 8cm, or about 7cm, and about 70cm to about 80cm, or about 74 to about 78cm, or about 76cm of water column head greater in the uncovered portion of the collection grid than gas pressure in the covered portion of the collection grid.

Preferably, a covered portion of a waste gas collection grid is provided with an adjustable control valve and wherein the automatic isolation valve is positioned between a vacuum pump and the adjustable control valve.

Preferably, the automatic isolation valve is operable to isolate a covered portion of a gas collection grid from an uncovered portion of a gas collection grid and prevent flow in either direction when isolating a covered portion and an uncovered portion of a gas collection grid.

Preferably, the gas collection system is for a waste pile having a first covered portion covered by an impermeable membrane and a second uncovered portion which is not covered by an impermeable membrane.

Preferably, the plurality of pressure relief valves comprise automatic pressure relief valves positioned in a covered portion of a waste pile for venting pressure from beneath an impermeable membrane.

Preferably, the plurality of pressure relief valves are spaced apart from one another between about one per 8000m2, approximately, and one per 4000m2, approximately, of a waste pile. Preferably, the plurality of pressure relief valves are spaced apart so that there are one or more per 4000m2, approximately, of a waste pile.

The plurality of pressure relief valves may be adapted to vent waste gas at pressures of between about 5cm to about 10cm, or about 6cm to about 8cm, or about 7cm, and about 70cm to about 80cm, or about 74 to about 78cm, or about 76cm of water column head.

Preferably, the automatic isolation valve is configured to isolate the covered portion of the waste gas collection grid when the vacuum pump stops operating.

An anti-lift topping may be provided and comprise synthetic turf positioned above a membrane and being adapted to resist up-lift of the membrane aerodynamically. An anti-lift topping may comprise synthetic turf positioned above the membrane and provided with a ballast. Also optionally, the synthetic turf can be provided with both aerodynamic anti-lift and ballast or even other anti-lift measures.

The specific techniques and structures employed to improve over the drawbacks of the prior systems and accomplish the advantages described herein will become apparent from the following detailed description of example embodiments and the appended drawings and claims.

BRIEF DESCRIPTION OF THE DRAWINGS

One or more example embodiments will now be described with reference to the accompanying Figures, in which:

Fig. 1 (prior art) is a schematic illustration of a prior art deep well arrangement for use at a landfill.

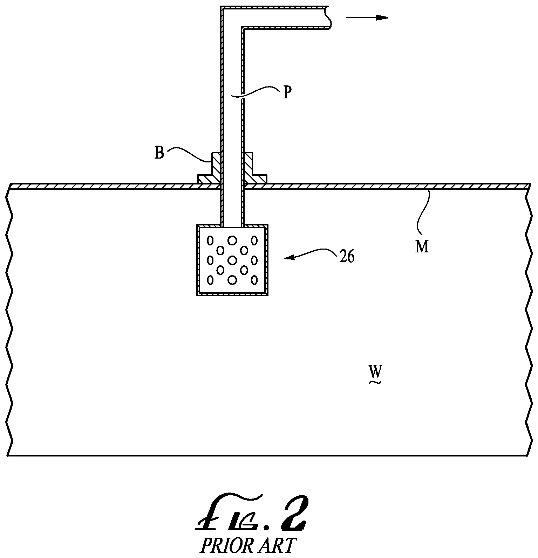

Fig. 2 (prior art) shows another prior art arrangement, this time showing a more shallow wellhead used to withdraw near-surface or sub-surface gas from beneath a membrane capping a waste pile.

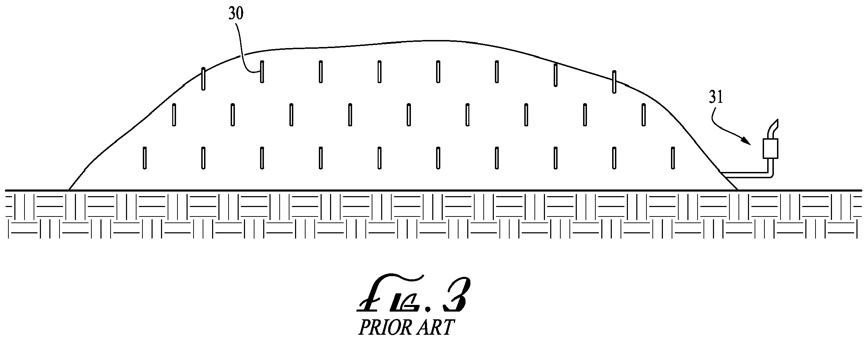

FIG. 3 (prior art) shows another prior art arrangement, this time depicting a landfill with multiple wellheads used to withdraw near-surface or sub-surface gas from beneath the surface

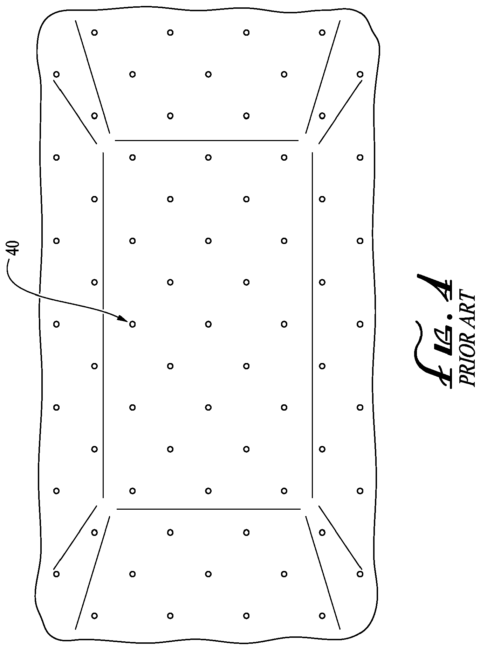

FIG. 4 (prior art) shows another prior art arrangement similar to that in FIG. 2, this time showing a field of wellheads spaced to extract the gases from a landfill and collect the same.

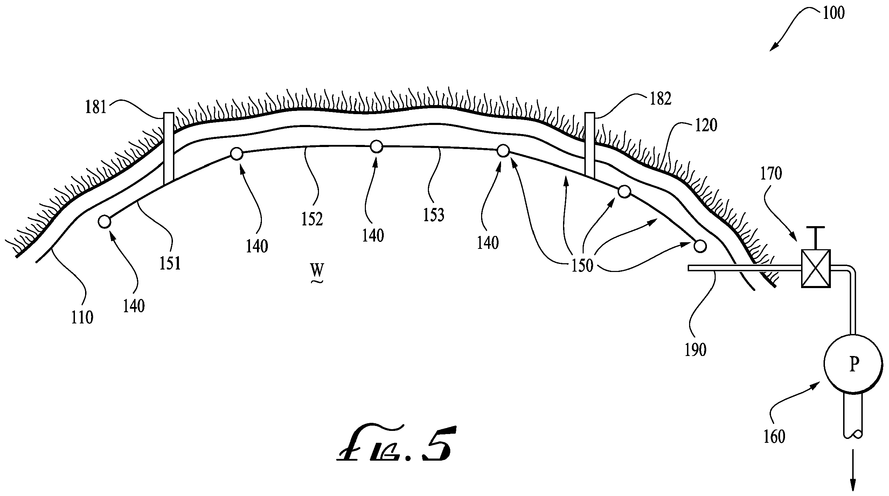

Fig. 5 is a schematic illustration of a fail-safe gas collection system according to a first example embodiment, for collecting waste gas from a waste pile having an impermeable membrane covering substantially all of the waste pile.

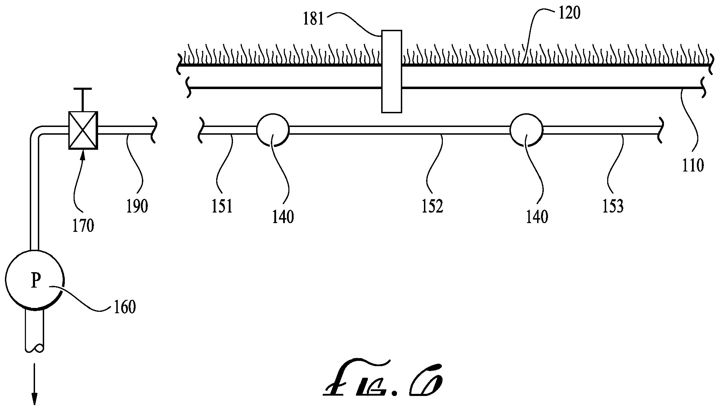

Fig. 6 is a schematic illustration of portions of the fail-safe gas collection system of Fig. 5.

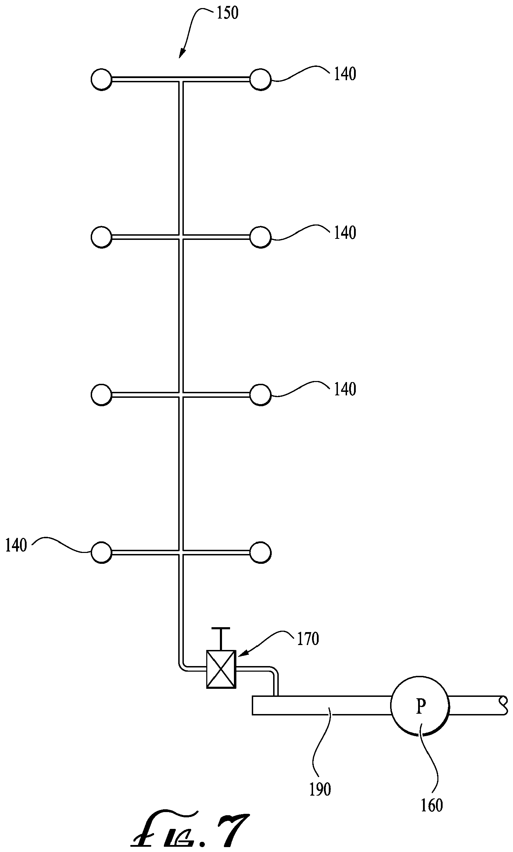

Fig. 7 is a schematic illustration of a portion of the fail-safe gas collection system of Fig. 5.

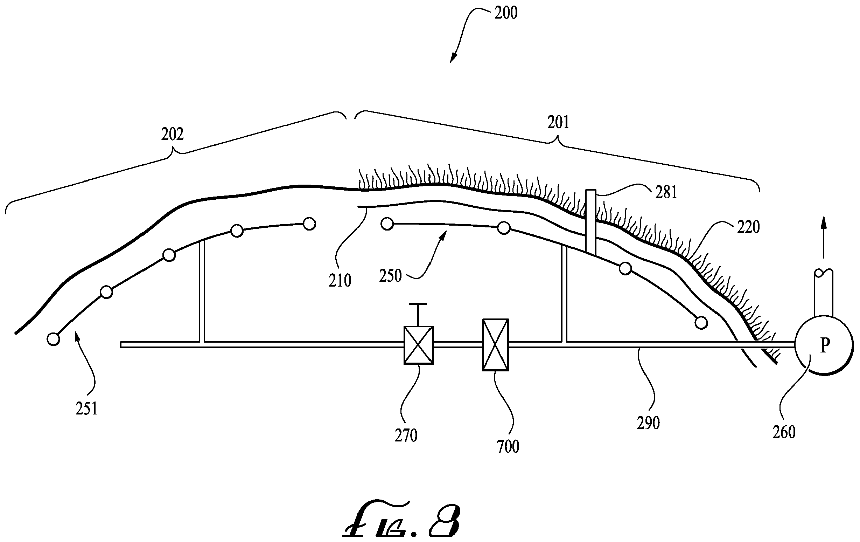

Fig. 8 is a schematic illustration of a fail-safe gas collection system according to a second example embodiment, for collecting waste gas from a waste pile having an impermeable membrane covering part, but not all, of the waste pile.

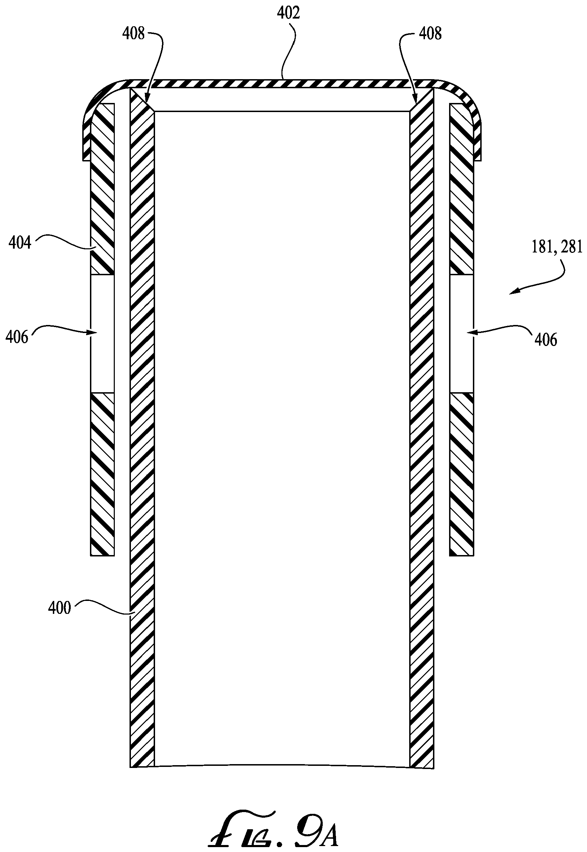

FIG. 9A is a schematic, sectional view of a portion of a gas vent or pressure relief valve according to an example embodiment of the present invention, shown with the valve in a closed position.

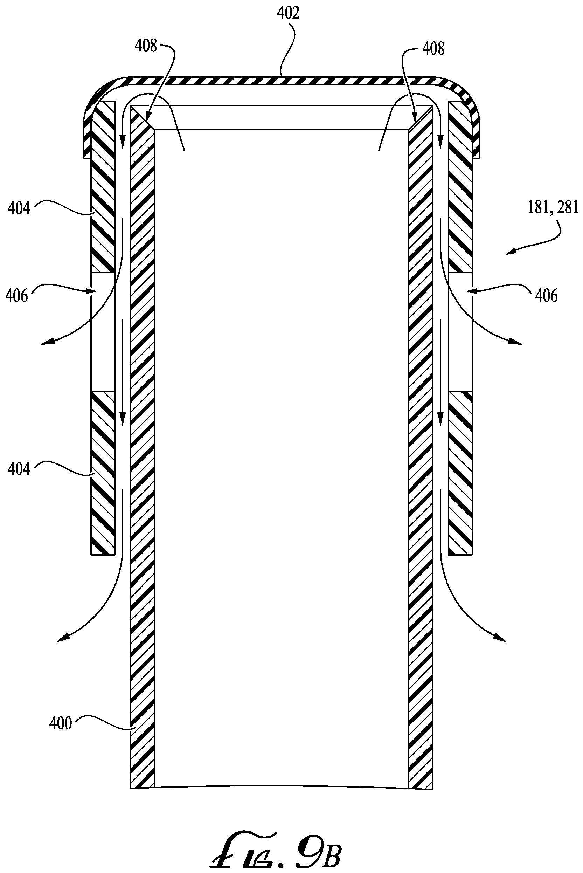

FIG. 9B is a schematic, sectional view of the gas vent or pressure relief valve of FIG. 9A, shown in an open position.

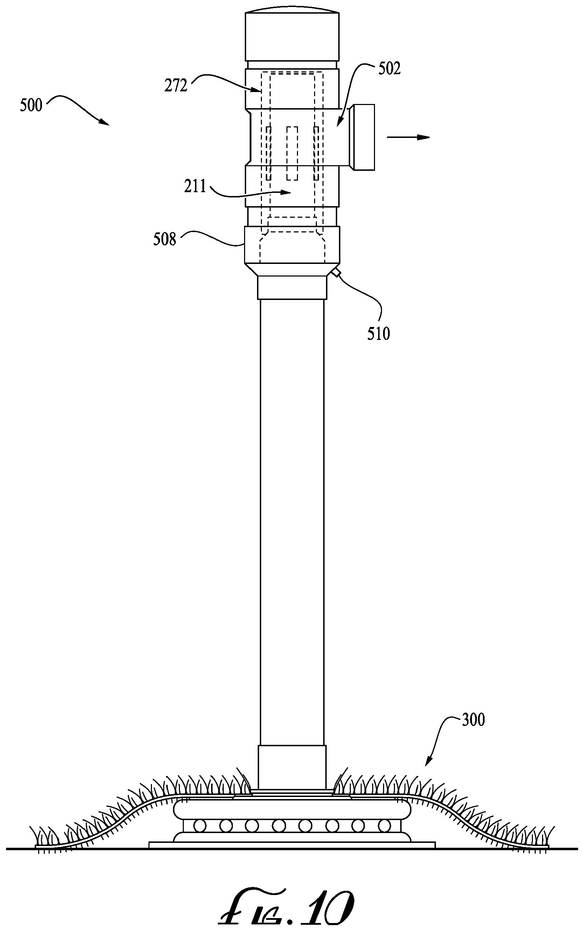

FIG. 10 is a schematic, elevation view of a gas vent or pressure relief valve for a landfill according to another example embodiment, having connectivity to a gas collection system and shown installed under a geomembrane.

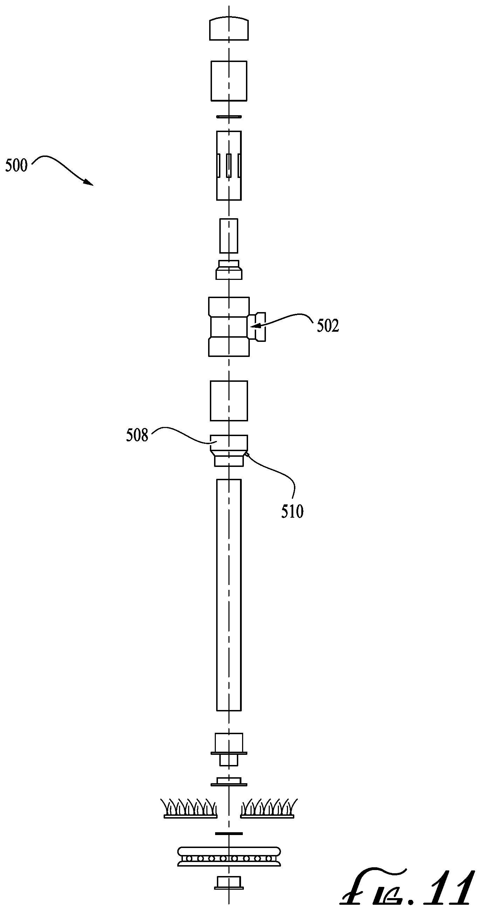

FIG. 11 is a schematic exploded view of gas vent for a landfill of FIG 10.

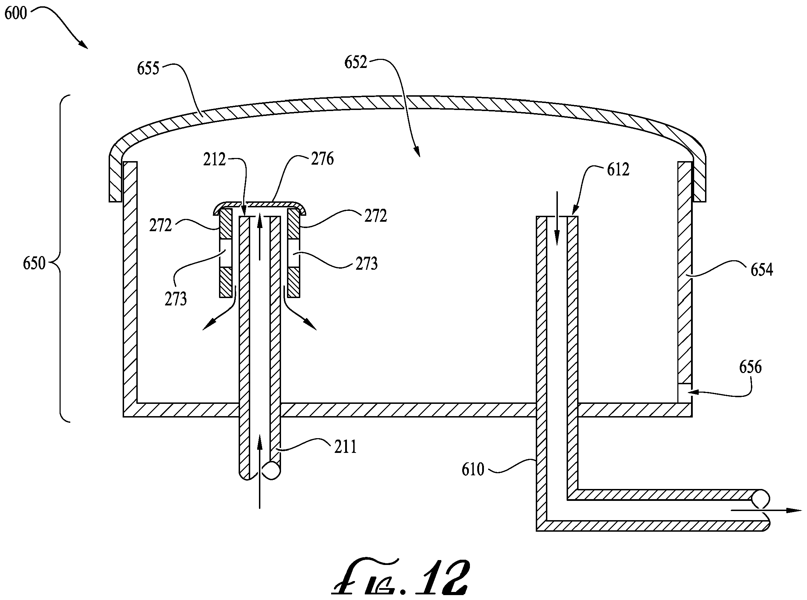

FIG. 12 is a schematic, sectional view of a portion of a gas vent or pressure relief valve for a landfill according to another example form, shown with the valve in an open position.

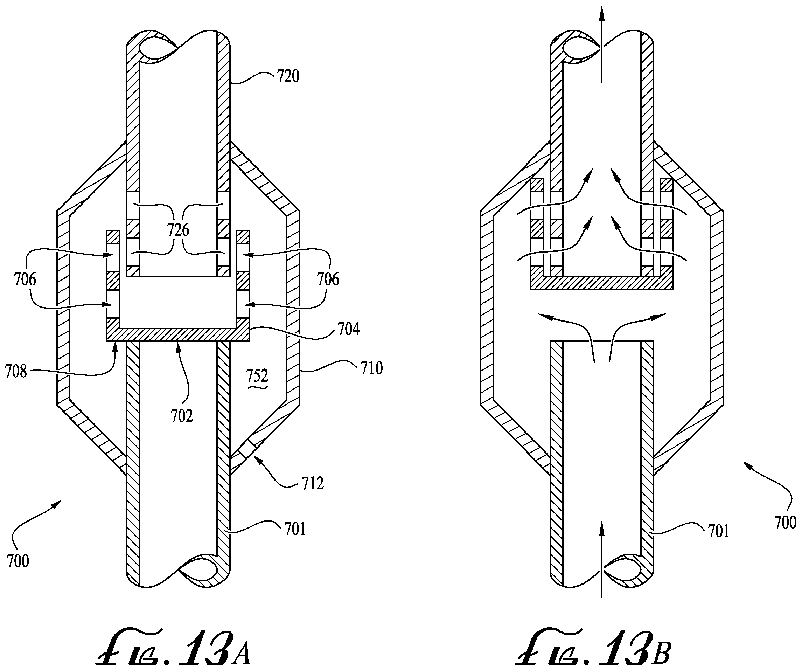

FIG. 13A is a schematic, sectional view of a portion of a gas vent or pressure relief valve for a landfill according to another example embodiment of the present invention, shown with the valve in a closed position.

FIG. 13B is a schematic, sectional view of the portion of a gas vent or pressure relief valve of FIG. 13A, shown with the valve in an open position.

DETAILED DESCRIPTION OF EXAMPLE EMBODIMENTS

Referring now in detail to Figs. 5-7, a first example embodiment of the present invention relates to gas collection systems for collecting waste gas from waste piles, such as from landfills. Fig. 5 shows a fail-safe gas collection system 100 for collecting waste gas from a waste pile W having an impermeable membrane 110 covering substantially all of the waste pile W. The fail-safe gas collection system 100 includes an anti-lift topping 120 for biasing the membrane 110 to remain in place in the presence of winds. A network or grid of conduits 150 is positioned beneath and extends beneath the membrane 110. A plurality of collection nodes 140 are positioned at spaced-apart positions on the network of conduits 150. A negative pressure pump 160 (such as a fan or vacuum pump) is provided for drawing gas through the collection nodes 140 of the network of conduits 150. An adjustable control valve 170 is positioned between the network of conduits 150 and the negative pressure pump 160. Pressure relief valves, such as valves 181, 182, etc., are provided to prevent the membrane 110 from ballooning in the event that the negative pressure pump 160 stops operating for a time.

Another example embodiment of the present invention as shown in Fig. 8 relates to a fail-safe waste gas collection system 200 for use with a waste pile W having a first covered portion 201 covered by an impermeable membrane 210 and a second uncovered portion 202 which is not covered by an impermeable membrane. The waste gas collection system 200 includes a waste gas collection grids 250, 251 for collecting waste gas from the covered portion 201 of the waste pile and the uncovered portion 202 of the waste pile, respectively. The fail-safe waste gas collection system 200 also includes a vacuum pump 260 connected to the waste gas collection grid 250 for drawing waste gas from the waste pile and at least one automatic pressure relief valve 281 positioned in the covered portion for venting pressure from beneath the membrane. An automatic isolation valve 700 positioned to isolate a covered portion 201 of the waste gas collection grid from an uncovered portion 202 of the waste gas collection grid to help prevent ballooning of the membrane 210 in the covered portion of the waste pile.

Preferably, the automatic isolation valve 700 is configured to isolate the covered portion 201 of the waste gas collection grid when the vacuum pump 260 stops operating. Preferably, the fail-safe gas collection system 200 includes an anti-lift topping 220 for biasing the membrane 210 to remain in place in the presence of winds. Preferably, the automatic isolation valve is operable to isolate the covered portion of the gas collection grid when the pressure in the uncovered portion of the gas collection grid is between about 5cm to about 10cm, or about 6cm to about 8cm, or about 7cm, and about 70cm to about 80cm, or about 74 to about 78cm, or about 76cm of water column head greater in the uncovered portion of the collection grid than gas pressure in the covered portion of the collection grid. Significantly, this isolation of the two parts of the collection grid helps to keep gas volume from the uncovered portion of the waste pile from being communicated to the covered portion of the waste pile, which could overwhelm the pressure relief valves' ability to vent the excess waste gas without ballooning the membrane. In this regard, the automatic isolation valve operates to protect the membrane from volume and pressure impacts from the uncovered portion of the waste pile while the vacuum pump is inoperable (such as during a power outage).

Preferably, the covered portion 201 of the waste gas collection grid is provided with an adjustable control valve 270 and wherein the automatic isolation valve 700 is positioned between the vacuum pump 260 and the adjustable control valve 270.

ANTI-LIFT TOPPING

In one optional form, the anti-lift topping 120, 220 can comprise synthetic turf positioned above the membrane and being adapted to resist up-lift of the membrane aerodynamically, but other anti-lift toppings can be employed. Preferably, the anti-lift topping comprises synthetic turf positioned above the membrane and can optionally be provided with a ballast. The geomembrane 110, 210 can be covered with a synthetic turf having synthetic strands that resemble grass with a pile height ranging from about 2cm to about 5cm depending on wind design velocities. The strands tend to break the laminar flow of the wind and also provide normal pressure on the geomembrane liner system when the strands bend, creating normal pressure. Preferably, the pile height is about 2cm to about 5cm or so. More preferably, the pile height is between about 2cm to 3cm and about 3 to 4cm.

Preferably, the synthetic strands are slender elongate elements. As used herein, "slender" indicates a length that is much greater than its transverse dimension(s). Examples of slender elongate elements contemplated as encompassed by embodiments of the present invention or in conjunction therewith are structures that resemble blades of grass, rods, filaments, tufts, follicle-like elements, fibers, narrow cone-shaped elements, etc. The synthetic strands extend upwardly from a base and form a mat or field of such strands. Such can simulate a field of grass, pine straw or similar. Moreover, while the anti-lift topping will be described below in connection with blade-like elements (grass blade-like, not necessarily like cutting blades) as an example embodiment, those skilled in the art will readily appreciate that the invention is not to be limited to the example embodiments.

Preferably, the chemical composition of the synthetic grass blades should be selected to be heat-resistant and UV-resistant (and to withstand exposure to sunlight, which generates heat in the blades and contains ultraviolet rays). Furthermore, the polymer yarns should not become brittle when subjected to low temperatures. The selected synthetic grass color and texture should be aesthetically pleasing. While various other materials may work well for the grass blades, it is presently believed that polyethylene fibers work best.

Optionally, the synthetic grass blades are tufted to have a density of between about 20 ounces/square yard and about 100 ounces/square yard. Preferably, the synthetic grass blades have a density of between about 20 and 40 ounces/square yard. The tufting is fairly homogeneous. In general, a "loop" is inserted at a gauge spacing to achieve the desired density. Each loop shows as two blades of grass at each tufted location. Preferably, the synthetic grass blades have a thickness of at least about 100 microns.

The synthetic grass blades are tufted into a geotextile backing. The geotextile backing preferably consists of one or more geotextiles made of polypropylene or polyethylene with UV stabilizers. The geotextiles can comprise slit film (tape yarn) or monofilament. Generally speaking, the lower the surface area of the yarn per unit weight of raw material, the better the ultraviolet (UV) performance. Monofilament geotextiles typically have a small cross section relative to their length, which inherently provides for a smaller surface exposed to UV light per unit weight of polypropylene or polyethylene. In other words, a yarn with a round cross-section typically will exhibit better UV resistance than a flat geometric shape.

Optionally, the geotextile backing can be a single layer backing, a double layer backing, or can have more than two layers. But it is preferred that a single layer or double layer backing be used. Optionally, the backing can be made of polypropylene or polyethylene. Also, optionally a separate membrane can be dispensed with, such as by applying a membrane-like layer to the back side of the synthetic geotextile. For example, a urethane coating can be sprayed onto the back of the synthetic geotextile and allowed to cure.

The prior art technique of using tarps or geomembranes to cover leach pads, landfills and stockpiles to protect the ore, waste and soil stockpiles from rain and weather damage typically requires substantial ballast or anchorage. This anti-lift synthetic turf allows the use of a membrane over large areas without requiring such ballast or anchorage. Instead, a synthetic cover layer 120 is provided that can resist wind uplift and thus protecting the impermeable geomembrane 110 below. The synthetic cover 120 contains grass-like filaments covering and protecting the impermeable geomembrane liner surface.

The inventive wind-resistant cover and liner was laboratory tested (at the Georgia Tech Research Institute ("GTRI") Wind Tunnel Lab) using wind tunnels to determine the uplift vertical pressures and shear pressures on the synthetic cover. The wind tunnel trials indicated that this novel cover resists the uplift forces of the wind. Minimal product weight of 0.3 lbs/sq-ft typically will be required to counteract the shear forces from the wind. Synthetic grass and geomembranes in the range of 30 to 40mil thickness would exceed this minimum weight-per-unit area threshold. The present inventors have confirmed the performance of this novel cover by testing the same as landfill covers for mines and general covers for ore stockpiles, dams, embankments, general stockpiles and the like.

During the wind tunnel investigation, the turf was experimentally evaluated for its aerodynamic properties and ballast requirements of the novel synthetic ground cover system under a range of wind speed conditions. The cover system was tested full scale in a subsonic model test facility wind tunnel wherein the normal forces loading (lbs/sq ft) and the shear stresses (lbs/sq ft) were determined for the proposed section of the materials (synthetic cover and geomembrane) as described herein.

Pressure variations across the height of a boundary layer were measured in the wind tunnel. Pressure variations are due to viscous forces. In order to investigate the unique characteristics of the flexible and permeable cover layer a traverse system was built into the model to actuate a pitot static probe vertically through the boundary layer. This allowed the measurement of the total and static pressure as a function of the probe height defined as h=0 at the upper surface of the geomembrane or geotextile backing. From these measurements the flow velocity was determined. This characterized the shape of the boundary layer that is by its nature a transition from the no slip condition at the surface (v=0) to the free stream.

A sophisticated 6-component force balance was utilized to measure the aerodynamic lift (L) and the total drag (D). These forces were transmitted to the balance through a vertical strut mounted underneath the model base. These forces represent the total sum of all pressure distribution acting on the model resolved vertically and tangentially as shown in the equation below:

$$L_{cover} = L - L_{amb} + L_{geotex/geome}$$

It was discovered that at the edges the wind subjects the synthetic turf to up to 89% of the total free stream. The blades are subject to higher velocities and thus higher increasing drag as the wind speed increases. The higher drag increases the bending of the blades back onto the backing geotextile(s). The effect of this has two counteracting impacts on the net lift. At lower velocities the synthetic blades are bent slightly with flow being deflected and accelerated over the edges. This flow acceleration increases the local velocity and lowers the local static pressure below that of the stream static, which creates the pressure differential building up with the associated uplift of the cover geotextiles. This force can be counteracted by building an anchor trench at the perimeter of the cover.

The wind-resistant synthetic turf creates a larger distance from the material surface to the "free stream" (free stream occurs where the wind flow is unaffected by the material). The cover radically breaks up the flow stream, increasing the boundary layer (distance from surface to free stream) to the point where uplift forces are very small. This is in stark contrast to a prior art exposed membrane cover, in which there is a minuscule distance from the surface (where velocity is 0 feet per second, which is the case for all materials and wind conditions) to free stream.

The boundary layer conditions are created by longer flow paths over a given surface and all boundaries grow in thickness and increase in turbulence with increasing distance. In the case of example embodiments of this invention, the interaction of the flow with the flexible blades causes the boundary layer growth to occur quite rapidly. It is also clearly seen in our experiments that little to no deflection occurred in the cover at a distance just over 15cm from the perimeter edge. The measured uplift results show values requiring minimal uplift resistance that can simply be achieved by the weight of the cover itself.

Exposed geomembrane covers have been used extensively in the past as covers for landfills and stockpiles in the solid waste and mining industries in order to prevent or minimize rainwater infiltration into the waste or the ore. In such prior art geomembrane applications, UV-resistance of the liner materials has not been a concern when HDPE and LLDPE, PVC materials are used as the plastic materials. For the synthetic grass used in embodiments of the present invention, the blades can be made of polyethylene, HDPE, LLDPE, PVC, or other UV-resistant material. While UV resistance is not an absolute requirement, it does provide an important long-term stability for the synthetic grass blades, adding to the overall performance of the system.

In addition to the wind-resistant synthetic turf described immediately above, the system 100, 200 can use ballast (such as sand, gravel, etc.) to help hold the membrane down. The ballast can be used over conventional synthetic turf or directly over the impermeable membrane 110, 210. Also, the anti-lift properties of the wind-resistant turf can be bolstered by adding ballast to the wind-resistant turf.

PRESSURE RELIEF VALVES

Preferably, the plurality of pressure relief valves (e.g., 181, 281) are adapted to vent waste gas at pressures of between about 5cm to about 10cm, or about 6cm to about 8cm, or about 7cm, and about 70cm to about 80cm, or about 74 to about 78cm, or about 76cm of water column head. Figs. 9A, 9B depict pressure relief valve 181, 281 including a canister 404 that is loosely fitted about and atop the upper portion 408 of a conduit 400. The conduit upper portion 408 has a pointed knife-like edge around the circumference. Gravity pulls the canister 404 downwardly atop the open end of the upper portion 408 and the contact between the pointed knife-like circumferential edge and the valve membrane 402 to create a normal seal against air being drawn into the conduit 400 from above. With the valve membrane 402 lifted slightly, as depicted in Fig. 9B, the gas under pressure in the conduit 400 can escape the conduit. The canister 404 is further depicted to have at least one, and preferably a pair of, apertures 406 along the side-wall. These apertures 406 can be slits, perforations or holes. As depicted in Fig. 9B, air escaping the conduit 400 can also be released through the apertures 406.

Figs. 10, 11 depict an alternate pressure relief valve 500 designed to function with a conduit and a canister similar to those described in previous examples,. The pressure relief valve 500 has a generally similar construction to the previously described version. The alternative pressure relief valve 500 includes a conduit or pipe 502 that is directed towards a gas destruction system. The gas destruction system can include a suction pump to actively remove the gas released through the conduit 211 and canister 272. Alternatively, or in combination, the gas destruction system can have a periodic-activated spark that burns the gas. The pressure relief valve 500 further includes a condensate drain plug 510 secured to a reducer 508 that connects the conduit 211 to the pressure relief valve similarly to the previously-described embodiment. The condensate drain plug 510 releases condensate collected within the reducer 508.

Fig. 12 depicts another alternative pressure relief valve 600 system designed to function with a conduit and a canister similar to those described in previous examples. Generally, pressure relief valve 600 is positioned adjacent an upper portion of the conduit 211 and defines a discharge chamber 652 therein into which gas from the discharge end 212 of the conduit is discharged, the discharge chamber being between the inside wall of the pressure relief valve 600 and the outside wall of the upper portion of the conduit 211. The pressure relief valve may include a base portion 654 and a weather-proof cap 655 as shown or the hood can be provided as a single component or piece. The pressure relief valve also includes an exhaust conduit 612 fluidly connected to the pressure relief valve and extending at least partially therein. Gas discharged into the discharge chamber 652 from conduit 211 is vented or exhausted through the exhaust conduit 612 which may be directed to atmosphere or to a destruction chamber as described above. The pressure relief valve 600 further includes a condensate drain hole or plug 656 which drains or releases condensate accumulated in the discharge chamber 652.

In example forms, the base portion 654 and cap 655 comprise a generally cylindrical profile. However, the base portion and cap may comprise other geometric profiles, such as for example polygonal or elliptical profiles, of various dimensions, and it is to be understood that the base portion 654 and cap 655 are not limited to any specific profile or size. Furthermore, the valve portion 270 comprising the canister 272 and seal 276 is operable as described herein notwithstanding a lack of in-line pressure within the discharge chamber 652. The seal 276 can be a rigid element, like a hard plastic or metal. Or in alternative forms, it can be a flexible membrane as described above in connection with prior figures.

Optionally, the pressure relief valves 181, 182 comprise a plurality of pressure relief valves spaced apart from one another at a spacing of between about one per 8000m2, approximately, and one per 4000m2, approximately. Optionally, the plurality of pressure relief valves are spaced about one per acre. Optionally, the plurality of pressure relief valves are positioned at least one or more per 4000m2, approximately.

Preferably, the plurality of pressure relief valves are adapted to vent waste gas at pressures of between about 5cm to about 10cm, or about 6cm to about 8cm, or about 7cm, and about 70cm to about 80cm, or about 74 to about 78cm, or about 76cm of water column head.

AUTOMATIC ISOLATION VALVE

Figs. 13A & 13B depict an automatic isolation valve 700 system designed to isolate one part of the waste gas collection system from another part of the gas collection system. In particular, the automatic isolation valve 700 isolates that portion of the gas collection system under the impermeable membrane from that portion which is not under the impermeable membrane. This isolation function is provided in response to pressure differentials between the two parts that may arise when power to the vacuum pump 160, 260 is lost.

The automatic isolation valve 700 is positioned adjacent an upper portion of the conduit 701 and defines a discharge chamber 752 therein into which gas from the discharge end of conduit 701 is discharged. The discharge chamber 752 is defined between the inside wall of the automatic isolation valve 700 and the outside wall of the upper portion of the conduit 210. Generally, the automatic isolation valve 700 comprises a cylindrical or generally bulbous profile wherein the inner diameter of the hood is larger than the outer diameter of the intake conduit 210. The intake conduit 701 connects to and extends at least partially into the automatic isolation valve 700 through a first end 710. At a second end, opposite the first end 710, the automatic isolation valve 700 system comprises an exhaust conduit 720 fluidly connecting the discharge chamber 752 to the surrounding atmosphere (or to a destruction system) as described above. In example forms, the exhaust conduit 720 is axially aligned with the intake conduit 701 and their respective two ends are separated by a gap or space therebetween. The automatic isolation valve 700 further includes a condensate drain hole or plug 712 which drains or releases condensate accumulated in the discharge chamber 752.

A valve portion or sleeve 708 is generally provided in the gap or space between the discharge end of the intake conduit 701 and intake end 722 of the exhaust conduit 720. Preferably, the valve portion 708 is substantially rigid and comprises a rigid perforated tube portion 704 and a rigid closed end portion 702. The valve portion 708 is loosely fitted about the intake end 722 of the exhaust conduit whereby the closed end portion is positioned between the intake and exhaust conduits. In example embodiments, the perforated tube portion 704 has a plurality of holes or apertures 706. The exhaust conduit 720 also includes a plurality of holes or apertures 726 near and about its intake end 722 wherein the plurality of holes 726 are configured to align with the plurality of holes 706 of the valve sleeve 708 when the valve sleeve is in an open position. According to example forms, the plurality of holes 706 of the valve sleeve 708 are arranged in two rows wherein the two rows are staggered or offset from one another, whereas the plurality of holes 726 are arranged in two rows that are uniformly or symmetrically positioned to one another. In other example forms, the plurality of holes 706 are uniformly or symmetrically patterned while the plurality of holes 726 are staggered or otherwise offset. The differing patterns ensures that the plurality of holes 706 and 726 are at least partially aligned even if the valve sleeve 708 rotates axially about the exhaust conduit 720. In some example forms, apertures 706 and 726 may comprise the same, or a combination of, shapes and/or configurations to ensure at least a partial alignment independent of the axial alignment of the valve sleeve 708 about the exhaust conduit 720. For example, the apertures 706 may be of elongated slots whereas apertures 726 are circular.

According to example forms, the automatic isolation valve 700 may be oriented substantially vertically or horizontally. The following applies when the automatic isolation valve 700 may be oriented substantially vertically. When the pressure in the intake conduit 701 is substantially equal to the pressure in the discharge chamber 752, the outer surface of the closed end portion 702 rests against the discharge end of the conduit 701, normally sealing the conduit against air being drawn into the conduit from above ground, as shown in Fig. 13A. When pressure in the conduit 701 is negative, this seal is made more positive and effective. When pressure in the conduit 701 is positive, the pressure beneath the closed end portion 702 (or in the intake conduit 701) gently lifts (or pushes) the valve sleeve 708 away from the discharge end 212 of the intake conduit 701 and thereby aligns the plurality of holes 706 of the valve sleeve 708 to the plurality of holes 726. With the closed end portion 702 offset from the intake conduit and plurality of holes 706 and 726 at least partially aligned, the gas under pressure in conduit 701 can escape into the discharge chamber 752, whereupon it is then vented to atmosphere, or a destruction system, through the exhaust conduit 720. If pressure in the exhaust conduit 720 should become greater than in the automatic isolation valve 700, the end portion 702 can be gently urged back toward the closed position of Fig. 10A.

If the automatic isolation valve 700 and the conduits 701 and 720 are arranged horizontally, the automatic isolation valve 700 operates substantially as described above, but without the force of gravity biasing the end 702 toward sealing against the open end of the conduit 701.

Another example embodiment of the present invention relates to a fail-safe waste gas collection system for use at a partly membrane-covered, partly uncovered waste pile having a waste gas collection grid connected to a vacuum pump. The fail-safe system includes a plurality of pressure relief valves to help protect the membrane against ballooning in the event that the vacuum pump stops operating. It also includes an automatic isolation valve to isolate a covered portion of the collection grid from an uncovered portion of the collection grid when gas pressure in the uncovered portion of the collection grid exceeds gas pressure in the covered portion of the collection grid.

Preferably, the automatic isolation valve 700 is operable to isolate the covered portion of the gas collection grid when the pressure in the uncovered portion of the gas collection grid is between about 5cm to about 10cm, or about 6cm to about 8cm, or about 7cm, and about 70cm to about 80cm, or about 74 to about 78cm, or about 76cm of water column head greater in the uncovered portion of the collection grid than gas pressure in the covered portion of the collection grid.

Preferably, the covered portion of the waste gas collection grid is provided with an adjustable control valve and wherein the automatic isolation valve 700 is positioned between the vacuum pump and the adjustable control valve.

Preferably, the automatic isolation valve 700 is operable to isolate the covered portion of the gas collection grid from the uncovered portion of the gas collection grid and prevent flow in either direction when isolating the two portions of the gas collection grid.

COLLECTION GRID

Preferably, the network of conduits 150 includes a grid of non-perforated pipes 151, 152, 153, etc., and the collection nodes include orifices positioned at distributed locations on the grid of non-perforated pipes (typically, where the pipes cross or intersect one another). A transport conduit 190 extends beneath the geomembrane 110, and without extending through the geomembrane 110, for transporting gas produced by the waste pile W. The transport conduit 190 can be smooth, corrugated, or part smooth and part corrugated. The transport conduit 190 can be of any desired shape. For example, it can be round or square. Alternatively, it can be a flat, wide, shallow pipe and can have internal ribs or other structure to keep the pipe from collapsing.

A similar transport conduit 290 is depicted in Fig. 8 and extends beneath the geomembrane 210, and without extending through the geomembrane 210, for transporting gas produced by the waste pile W.

Preferably, the collection grid is substantially cruciform in shape and at intersections of various conduits an adapter T or cross is provided. Preferably, the grid includes at least one trunk conduit and the at least two branch conduits each comprise an elongate, non-perforated outer cover. Optionally, the at least four collector conduits each comprise an elongate perforated outer cover.

The sub-surface gas to be collected and withdrawn with example embodiments of the present invention can be any of several sub-surface gases, such as natural gas. The fluid collected and conveyed can be gaseous or liquid or a mixture of the two.

The gas collection grid can include a graduated conveyance grid with at least one high-volume, low-profile fluid trunk conduit. It also can include at least two medium-volume, low-profile fluid branch conduits connected to and feeding into the at least one high-volume, low profile trunk conduit. Further, it can include at least four lower-volume, low-profile collector conduits connected to and feeding into the at least two medium-volume, low profile branch conduits, with each branch conduit being connected to at least two of the collector conduits. With this construction, gas can be drawn into the smaller collector conduits, gathered into the somewhat larger branch conduits, and finally into the trunk conduit.

Preferably, the grid is substantially cruciform in shape and at intersections of various conduits an adapter T or cross is provided. Optionally, the adapter T or cross has an upper opening and is provided with a cover for covering the upper opening.

Preferably, the grid includes at least one trunk conduit and the at least two branch conduits each comprise an elongate, non-perforated outer cover. Optionally, the at least four collector conduits each comprise an elongate perforated outer cover. Preferably, the elongate outer cover comprises a polymer.

The system design, coupled with a membrane cover, creates a superior barrier and conveyance system for gas emissions. The radius of influence of the system can be every square foot between the waste mass and the atmosphere.

The shallow gas well collection system is designed specifically for both long and short-term interim cover areas. The system creates a semi-conical radius of influence into the waste mass and a linear radius of influence between the impermeable membrane and protective cover layer. The shallow gas well system creates barriers of both an impermeable membrane and vacuum in an area of the landfill that is critical to preventing fugitive emission from escaping into the environment.

The system alleviates the high costs and maintenance associated with deep well gas collection designs and the low performance characteristics of horizontal collectors as well as the multiple membrane penetrations associated with both deep well and surficial gas collection systems. To accomplish this, a radius of influence across a large area is created by ensuring an available vacuum pressure is sustained throughout the shallow gas well gas collection system with an overlying impermeable membrane. This is achieved by utilizing the site-specific gas generation rate modeling to determine how much gas will need to be collected from an area. The estimated gas volume and the collection area size are then calculated along with the available vacuum, industry standards for friction loss in gas conveyance to determine the size of each orifice in the shallow gas well's flux chamber.

Preferably, there is no need for overlying membrane penetrations at the individual shallow gas well gas collection points. Preferably, each gas collection point has a flux chamber that penetrates through the protective soil layer and onto the top of the underlying waste mass. The flux chamber is made up of a larger diameter solid outer pipe with smaller diameter perforated pipe. The area between the two is filled with a highly permeable material such as gravel. The interior portion of the smaller diameter is where both the orifice, collector and condensate drain reside.

It is to be understood that this invention is not limited to the specific devices, methods, conditions, or parameters of the example embodiments described and/or shown herein, and that the terminology used herein is for the purpose of describing particular embodiments by way of example only. Thus, the terminology is intended to be broadly construed and is not intended to be unnecessarily limiting of the claimed invention. For example, as used in the specification including the appended claims, the singular forms "a," "an," and "the" include the plural, the term "or" means "and/or," and reference to a particular numerical value includes at least that particular value, unless the context clearly dictates otherwise. In addition, any methods described herein are not intended to be limited to the sequence of steps described but can be carried out in other sequences, unless expressly stated otherwise herein.

While the claimed invention has been shown and described in example forms, it will be apparent to those skilled in the art that many modifications, additions, and deletions can be made therein without departing from the spirit and scope of the invention as defined by the following claims.

## Claims

1. A fail-safe waste gas collection system for a waste pile having a membrane-covered portion and an uncovered portion, the waste pile having a waste gas collection grid extending to both the membrane-covered portion of the waste pile and the uncovered portion of the waste pile, and a vacuum pump connected to the waste gas collection grid, wherein the fail-safe waste gas collection system comprises:

   a plurality of pressure relief valves to help protect a membrane against ballooning in the event that a vacuum pump stops operating; and
   an automatic isolation valve to isolate a membrane-covered portion of a collection grid from an uncovered portion of a collection grid when a gas pressure in an uncovered portion of a collection grid exceeds a gas pressure in a membrane-covered portion of a collection grid.

2. A fail-safe waste gas collection system as claimed in Claim 1, wherein the automatic isolation valve is operable to isolate a covered portion of a gas collection grid when a pressure in an uncovered portion of a gas collection grid is between about 1cm about 80cm of water column head greater in an uncovered portion of a collection grid than a gas pressure in a covered portion of a collection grid.

3. A fail-safe waste gas collection system as claimed in Claim 1 or 2, wherein a covered portion of a waste gas collection grid is provided with an adjustable control valve and wherein the automatic isolation valve is positioned between a vacuum pump and the adjustable control valve.

4. A fail-safe waste gas collection system as claimed in any of Claims 1 to 3, wherein the automatic isolation valve is operable to isolate a covered portion of a gas collection grid from an uncovered portion of a gas collection grid and prevent flow in either direction when isolating a covered portion and an uncovered portion of a gas collection grid.

5. A fail-safe waste gas collection system as claimed in any of the preceding Claims, for a waste pile having a first covered portion covered by an impermeable membrane and a second uncovered portion which is not covered by an impermeable membrane.

6. A fail-safe waste gas collection system as claimed in Claim 5, wherein the plurality of pressure relief valves comprises automatic pressure relief valves positioned in a covered portion of a waste pile for venting pressure from beneath an impermeable membrane.

7. A fail-safe waste gas collection system as claimed in any of the preceding Claims, wherein the automatic isolation valve is configured to isolate a covered portion of a waste gas collection grid when a vacuum pump stops operating.

*FIG.1*
*PRIOR ART*

FIG. 2
PRIOR ART

**FIG.3**
*PRIOR ART*

FIG. 4
PRIOR ART

Fig. 5

Fig. 6

150

140

140

140

140

170

190

160

P

FIG. 7

Fig. 8

FIG. 9A

*FIG. 9B*

Fig.10

Fig.11

EP 4 309 812 A1

*Fig.12*

Fig. 13B

Fig. 13A

**EP 4 309 812 A1**

Europäisches
Patentamt

European
Patent Office

Office européen
des brevets

# EUROPEAN SEARCH REPORT

Application Number

EP 23 18 5685

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 2006/034664 A1 (AUGENSTEIN DON C [US] ET AL) 16 February 2006 (2006-02-16) * the whole document * | 1-7 | INV. B09B1/00 |
| A,P | ABEDI MOHAMMADMAHDI ET AL: "Smart Geosynthetics and Prospects for Civil Infrastructure Monitoring: A Comprehensive and Critical Review", INSTITUTE FOR SUSTAINABILITY AND INNOVATION IN STRUCTURAL ENGINEERING, SCHOOL OF ENGINEERING, UNIVERSITY OF MINHO, CAMPUS DE AZURÉM, 4800-058 GUIMARÃES, PORTUGAL, vol. 15, no. 12, 8 June 2023 (2023-06-08), page 9258, XP093102017, DOI: 10.3390/su15129258 * the whole document * | 1-7 | |

TECHNICAL FIELDS SEARCHED (IPC)

B09B

The present search report has been drawn up for all claims

| Place of search | Date of completion of the search | Examiner |
|---|---|---|
| Munich | 15 November 2023 | Dupuis, Jean-Luc |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

25

## ANNEX TO THE EUROPEAN SEARCH REPORT
## ON EUROPEAN PATENT APPLICATION NO.

EP 23 18 5685

This annex lists the patent family members relating to the patent documents cited in the above-mentioned European search report.
The members are as contained in the European Patent Office EDP file on
The European Patent Office is in no way liable for these particulars which are merely given for the purpose of information.

15-11-2023

| Patent document cited in search report | | Publication date | Patent family member(s) | | Publication date |
|---|---|---|---|---|---|
| US 2006034664 | A1 | 16-02-2006 | US | 2006034664 A1 | 16-02-2006 |
| | | | US | 2007243023 A1 | 18-10-2007 |
| | | | WO | 2006010164 A2 | 26-01-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20060034664 A